# EUROPEAN PATENT APPLICATION

(11) **EP 4 410 835 A1**
(43) Date of publication of application: **07.08.2024**
(21) Application number: 22875004.8
(22) Date of filing: 28.09.2022
(51) Int. Cl.: C07K 16/28, C12N 15/85, A61K 39/395, A61P 35/00

(54) **ANTI-LAG3 ANTIBODY, PHARMACEUTICAL COMPOSITION AND USE**

(30) Priority: 29.09.2021 CN 202111149114
(71) Applicant: Akeso Biopharma, Inc., Zhongshan, Guangdong 528437 (CN)
(72) Inventor: XIA, Yu, Zhongshan, Guangdong 528437 (CN); WANG, Zhongmin, Zhongshan, Guangdong 528437 (CN); ZHANG, Peng, Zhongshan, Guangdong 528437 (CN); LI, Baiyong, Zhongshan, Guangdong 528437 (CN)
(74) Representative: Berggren Oy
(86) International application number: PCT/CN2022/122185
(87) International publication number: WO 2023/051621

(57) **Abstract**

The present invention belongs to the field of biomedicine, and relates to an anti-LAG3 antibody, a pharmaceutical composition containing same, and the use thereof. Specifically, the present invention relates to an anti-LAG3 antibody or an antigen binding fragment thereof, wherein the antibody comprises a heavy chain variable region and a light chain variable region, the heavy chain variable region comprises HCDR1-HCDR3 having amino acid sequences as shown in SEQ ID NOs: 9-11, respectively, and the light chain variable region comprises LCDR1-LCDR3 having amino acid sequences as shown in SEQ ID NOs: 12-14, respectively. The anti-LAG3 antibody of the present invention has superior affinity and specificity, and has good application prospects.

## Description

### TECHNICAL FIELD

The present invention belongs to the field of biomedicine, and relates to an anti-LAG3 antibody, a pharmaceutical composition comprising same, and use thereof.

### BACKGROUND

Tumor, especially a malignant tumor, is a serious health-threatening disease in the world today, and it is the second leading cause of death among various diseases. In recent years, the incidence of the disease has been increasing remarkably. The malignant tumor is characterized by poor treatment response, high late metastasis rate, and poor prognosis. Although conventional treatment methods (such as radiotherapy, chemotherapy, and surgical treatment) adopted clinically at present alleviate the pain to a great extent and prolong the survival time, the methods have great limitations, and it is difficult to further improve their efficacy.

Lymphocyte-activation gene 3 (LAG3), namely CD223, is a type I transmembrane protein composed of 498 amino acids and is a member of the immunoglobulin superfamily (IgSF). LAG3 is mainly expressed in activated CD4⁺ T cells and CD8⁺ T cells. Additionally, in cells such as natural killer (NK) cells, B cells, regulatory T cells (Tregs), and plasmacytoid dendritic cells (pDCs), LAG3 is also expressed. (Ruffo Elisa, Wu Richard C, Bruno Tullia C et al., Lymphocyte-activation gene 3 (LAG3): The next immune checkpoint receptor. [J].Semin Immunol, 2019, 42: 101305.).

The LAG3 molecule gene is located on human chromosome 12 (20p13.3), adjacent to the CD4 molecule gene, and both have the same exons and introns. The LAG3 molecule and the CD4 molecule have a high degree of structural similarity, although the amino acid sequence homology between the two is only about 20%. Major histocompatibility complex class II (MHC II) molecules, liver sinusoidal endothelial cell lectin (LSECtin) molecules, and galectin-3 molecules are related ligands for the LAG3 molecule. The MHC class II molecules are the main ligands for LAG3. The affinity (Kd: 60 nmol·L⁻¹) of LAG3 molecules for the MHC class II molecules is 100 times that of CD4 molecules, indicating that the LAG3 molecules can effectively compete with the CD4 molecules for binding to the MHC class II molecules and inhibit T cell activation.

In the tumor microenvironment, the expression of the immunosuppressive molecule LAG3 can be detected 24 hours after T cell activation, which then leads to T cell dysfunction or apoptosis. The LAG3 molecule, through its D1 domain (which contains one proline-rich loop structure), forms a dimer molecule to specifically bind to the MHC class II molecule in the first signaling axis of CD4⁺ T cell activation, "CD3-TCR-MHCII", so that on one hand, a signal transduction pathway for T cell activation is blocked, and on the other hand, an intracellular segment of the LAG3 molecule (HIEELE motif) generates an immunosuppressive signal to down-regulate the activity of CD4⁺ T cells. The LAG3 molecule can promote the differentiation of Treg cells, participate in downstream signaling of signal transducer and activator of transcription 5, and thus enhance the inhibitory effect of Treg cells, which is one of the mechanisms by which tumors escape from killing by the immune system (Andrews Lawrence P, Marciscano Ariel E, Drake Charles G, et al., LAG3 (CD223) as a cancer immunotherapy target. [J]. Immunol Rev, 2017, 276: 80-96.).

Multiple studies have shown that LAG3 is overexpressed in tumor-infiltrating CD8⁺ T cells of various malignant tumors. For example, in ovarian cancer, tumor-infiltrating New York esophageal squamous cell carcinoma 1 (NY-ESO-1) antigen-specific CD8⁺ T cells express high levels of PD-1 and LAG3, and have a reduced ability to produce IFN-γ and TNF-α, thereby leading to lymphocyte inactivation. Galectin-3 and LSECtin interact primarily with LAG3 to regulate the activation and function of CD8⁺ T cells. In addition, melanoma antigen-specific T cells isolated from patients with metastatic melanoma exhibit a significant up-regulation in the expression of LAG3 and other immune checkpoint molecules CTLA-4 and TIM-3. (Liu Hao, Li Xinying, Luo Longlong, et al., Research advances in biological function of lymphocyte activation gene-3 (LAG-3) molecule and clinical application of antibody drugs targeting LAG-3 [J]. Chinese Journal of Pharmacology and Toxicology, 2019, 33(01): 70-78.). Currently, a plurality of LAG3 antibody medicaments have entered the clinical research stages, among which Bristol Myers Squibb's Relatlimab has the fastest progress, with 10 clinical studies underway. The vast majority of these studies involve the combination therapy of Relatlimab with nivolumab, used for the treatment of tumors such as hematological malignancies, melanoma, glioblastoma, renal cell carcinoma, non-small cell lung cancer, and the like.

There is currently a need to develop a novel anti-LAG3 antibody medicament.

### SUMMARY

Through intensive studies and creative efforts, the inventors have obtained an anti-LAG3 antibody. The inventors have surprisingly found that the anti-LAG3 antibody of the present invention (also referred to as the antibody or the antibody of the present invention for short) has superior affinity and/or specificity, and is even superior in one or more respects compared to positive control antibodies (e.g., Relatlimab). The present invention is detailed below.

One aspect of the present invention relates to an anti-LAG3 antibody or an antigen-binding fragment thereof, comprising a heavy chain variable region and a light chain variable region, wherein
the heavy chain variable region comprises HCDR1-HCDR3 having amino acid sequences set forth in SEQ ID NOs: 9-11, respectively, and the light chain variable region comprises LCDR1-LCDR3 having amino acid sequences set forth in SEQ ID NOs: 12-14, respectively; the heavy chain variable region comprises HCDR1-HCDR3 having amino acid sequences set forth in SEQ ID NOs: 9-11, respectively, and the light chain variable region comprises LCDR1-LCDR3 having amino acid sequences set forth in SEQ ID NO: 12, SEQ ID NO: 15 and SEQ ID NO: 16, respectively;
   or
the heavy chain variable region comprises HCDR1-HCDR3 having amino acid sequences set forth in SEQ ID NOs: 9-11, respectively, and the light chain variable region comprises LCDR1-LCDR3 having amino acid sequences set forth in SEQ ID NO: 17, SEQ ID NO: 15 and SEQ ID NO: 14, respectively.

In some embodiments of the present invention, the antibody or the antigen-binding fragment thereof is provided, wherein
the heavy chain variable region of the antibody has an amino acid sequence set forth in SEQ ID NO: 2, and the light chain variable region of the antibody has an amino acid sequence set forth in SEQ ID NO: 4;
the heavy chain variable region of the antibody has an amino acid sequence set forth in SEQ ID NO: 2, and the light chain variable region of the antibody has an amino acid sequence set forth in SEQ ID NO: 6;
   or
the heavy chain variable region of the antibody has an amino acid sequence set forth in SEQ ID NO: 2, and the light chain variable region of the antibody has an amino acid sequence set forth in SEQ ID NO: 8.

In some embodiments of the present invention, the antibody or the antigen-binding fragment thereof is provided, wherein the antibody or the antigen-binding fragment thereof is selected from a Fab, a Fab', an F(ab')₂, an Fd, an Fv, a dAb, a complementarity determining region fragment, a single chain fragment variable, a humanized antibody, or a chimeric antibody. In some embodiments of the present invention, the antibody or the antigen-binding fragment thereof is provided, wherein the antibody binds to human LAG3-mFc with an EC₅₀ of less than 0.2 nM, such as less than 0.15 nM, less than 0.1 nM, less than 0.08 nM, 0.06 nM, or less than 0.05 nM, or less; preferably, the EC₅₀ value is determined by indirect ELISA.

In some embodiments of the present invention, the antibody or the antigen-binding fragment thereof is provided, wherein
the antibody comprises a non-CDR region derived from a species other than murine, such as from a human antibody.

In some embodiments of the present invention, the antibody or the antigen-binding fragment thereof is provided, wherein
the antibody comprises a constant region derived from a human antibody;
preferably, the constant region of the antibody is selected from constant regions of human IgG1, IgG2, IgG3 or IgG4.

In some embodiments of the present invention, the antibody or the antigen-binding fragment thereof is provided, wherein
a heavy chain constant region of the anti-LAG3 antibody is Ig gamma-1 chain C region (e.g., as set forth in SEQ ID NO: 18) or Ig gamma-4 chain C region (e.g., as set forth in SEQ ID NO: 20), and a light chain constant region of the anti-LAG3 antibody is Ig kappa chain C region (e.g., as set forth in SEQ ID NO: 19).

In some embodiments of the present invention, the anti-LAG3 antibody is a monoclonal antibody.

In some embodiments of the present invention, the anti-LAG3 antibody is in an immunoglobulin form.

In some embodiments of the present invention, the anti-LAG3 antibody is a single chain fragment variable.

Another aspect of the present invention relates to an antibody-drug conjugate (ADC), comprising an antibody or an antigen-binding fragment thereof and a small molecule drug, wherein the antibody or the antigen-binding fragment thereof is the anti-LAG3 antibody or the antigen-binding fragment thereof according to any embodiment of the present invention; preferably, the small molecule drug is a small molecule cytotoxic drug; and more preferably, the small molecule drug is an anti-tumor chemotherapeutic drug.

The chemotherapeutic drug may be a conventional anti-tumor chemotherapeutic drug, such as an alkylating agent, an antimetabolite, an anti-tumor antibiotic, a plant-based anticancer agent, a hormone, and an immunological agent.

In one or more embodiments of the present invention, the antibody-drug conjugate is provided, wherein the antibody or the antigen-binding fragment thereof is linked to the small molecule drug via a linker; the linker may be one known to those skilled in the art, for example, a hydrazone bond, a disulfide bond, or a peptide bond.

In one or more embodiments of the present invention, the antibody-drug conjugate is provided, wherein the molar ratio of the antibody or the antigen-binding fragment thereof to the small molecule drug is 1:(2-4), e.g., 1:2, 1:3, or 1:4.

Yet another aspect of the present invention relates to an isolated nucleic acid molecule encoding the anti-LAG3 antibody according to any embodiment of the present invention. Yet another aspect of the present invention relates to a recombinant vector comprising the isolated nucleic acid molecule of the present invention.

Yet another aspect of the present invention relates to a host cell comprising the isolated nucleic acid molecule of the present invention or the recombinant vector of the present invention.

Yet another aspect of the present invention relates to a method for preparing the antibody or the antigen-binding fragment thereof according to any embodiment of the present invention, comprising: culturing the host cell of the present invention in a suitable condition, and isolating the antibody or the antigen-binding fragment thereof from the cell cultures.

Yet another aspect of the present invention relates to a pharmaceutical composition comprising the antibody or the antigen-binding fragment thereof according to any embodiment of the present invention, or the antibody-drug conjugate according to any embodiment of the present invention, wherein optionally, the pharmaceutical composition further comprises a pharmaceutically acceptable auxiliary material.

Yet another aspect of the present invention relates to use of the antibody or the antigen-binding fragment thereof according to any embodiment of the present invention, or the antibody-drug conjugate according to any embodiment of the present invention in the preparation of a medicament for treating and/or preventing a tumor or anemia, wherein preferably, the tumor is selected from one or more of ovarian cancer, esophageal cancer, melanoma, a hematological malignancy, glioblastoma, renal cell carcinoma, lung cancer, prostate cancer, bladder cancer, colon cancer, rectal cancer, liver cancer, gastrointestinal cancer, breast cancer, brain cancer, pancreatic cancer, thyroid cancer, head and neck cancer, and kidney cancer;
preferably, the lung cancer is non-small cell lung cancer;
preferably, the hematological malignancy is leukemia;
preferably, the esophageal cancer is esophageal squamous cancer.

The antibody or the antigen-binding fragment thereof according to any embodiment of the present invention, or the antibody-drug conjugate according to any embodiment of the present invention is for use in treating and/or preventing a tumor or anemia, wherein preferably, the tumor is selected from one or more of ovarian cancer, esophageal cancer, melanoma, a hematological malignancy, glioblastoma, renal cell carcinoma, lung cancer, prostate cancer, bladder cancer, colon cancer, rectal cancer, liver cancer, gastrointestinal cancer, breast cancer, brain cancer, pancreatic cancer, thyroid cancer, head and neck cancer, and kidney cancer;
preferably, the lung cancer is non-small cell lung cancer;
preferably, the hematological malignancy is leukemia;
preferably, the esophageal cancer is esophageal squamous cancer.

Yet another aspect of the present invention relates to a method for treating and/or preventing a tumor or anemia, comprising a step of administering to a subject in need an effective amount of the antibody or the antigen-binding fragment thereof according to any embodiment of the present invention, or the antibody-drug conjugate according to any embodiment of the present invention, wherein
preferably, the tumor is selected from one or more of ovarian cancer, esophageal cancer, melanoma, a hematological malignancy, glioblastoma, renal cell carcinoma, lung cancer, prostate cancer, bladder cancer, colon cancer, rectal cancer, liver cancer, gastrointestinal cancer, breast cancer, brain cancer, pancreatic cancer, thyroid cancer, head and neck cancer, and kidney cancer;
preferably, the lung cancer is non-small cell lung cancer;
preferably, the hematological malignancy is leukemia;
preferably, the esophageal cancer is esophageal squamous cancer.

In the present invention, unless otherwise defined, the scientific and technical terms used herein have the meanings generally understood by those skilled in the art. In addition, the laboratory operations of cell culture, molecular genetics, nucleic acid chemistry and immunology used herein are the routine procedures widely used in the corresponding fields. Meanwhile, in order to better understand the present invention, the definitions and explanations of the relevant terms are provided below.

As used herein, the term EC₅₀ refers to the concentration for 50% of maximal effect, i.e., the concentration that can cause 50% of the maximal effect.

As used herein, the term "antibody" refers to an immunoglobulin molecule that generally consists of two pairs of polypeptide chains (each pair with one "light" (L) chain and one "heavy" (H) chain). Antibody light chains are classified into κ and λ light chains. Heavy chains are classified into µ, δ, γ, α, or ε. Isotypes of antibodies are defined as IgM, IgD, IgG, IgA, and IgE. In light chains and heavy chains, the variable region and constant region are linked by a "J" region of about 12 or more amino acids, and the heavy chain further comprises a "D" region of about 3 or more amino acids. Each heavy chain consists of a heavy chain variable region (VH) and a heavy chain constant region (CH). The heavy chain constant region consists of 3 domains (CH1, CH2, and CH3). Each light chain consists of a light chain variable region (VL) and a light chain constant region (CL). The light chain constant region consists of one domain CL. The constant region of the antibody can mediate the binding of immunoglobulins to host tissues or factors, including the binding of various cells of the immune system (e.g., effector cells) to the first component (C1q) of the classical complement system. The VH and VL regions can be further subdivided into hypervariable regions (called complementarity determining regions (CDRs)), between which conservative regions called framework regions (FRs) are distributed. Each VH and VL consists of 3 CDRs and 4 FRs arranged from amino terminus to carboxyl terminus in the following order: FR1, CDR1, FR2, CDR2, FR3, CDR3, and FR4. The variable regions (VH and VL) of each heavy chain/light chain pair form an antibody-binding site. The assignment of amino acids to the regions or domains is based on Bethesda M.d., Kabat Sequences of Proteins of Immunological Interest (National Institutes of Health, (1987 and 1991)), or Chothia & Lesk J. Mol. Biol, 1987; 196: 901-917; Chothia et al., Nature, 1989; 342: 878-883, or the definition of the IMGT numbering system, see the definition in Ehrenmann F, Kaas Q, Lefranc M P., IMGT/3Dstructure-DB and IMGT/DomainGapAlign: a database and a tool for immunoglobulins or antibodies, T cell receptors, MHC, IgSF and MhcSF[J]., Nucleic acids research, 2009; 38(suppl_1): D301-D307.

The term "antibody" is not limited by any specific method for producing the antibody. For example, the antibody includes a recombinant antibody, a monoclonal antibody, and a polyclonal antibody. The antibody may be antibodies of different isotypes, such as IgG (e.g., subtype IgG1, IgG2, IgG3, or IgG4), IgA1, IgA2, IgD, IgE, or IgM.

As used herein, the terms "mAb" and "monoclonal antibody" refer to an antibody or a fragment of an antibody that is derived from a group of highly homologous antibodies, i.e., from a group of identical antibody molecules, except for natural mutations that may occur spontaneously. The monoclonal antibody is highly specific for a single epitope on an antigen. The polyclonal antibody, relative to the monoclonal antibody, generally comprises at least 2 or more different antibodies which generally recognize different epitopes on an antigen. Monoclonal antibodies can generally be obtained using hybridoma technology first reported by Kohler et al. (Köhler G, Milstein C. Continuous cultures of fused cells secreting antibody of predefined specificity [J]. Nature, 1975; 256(5517): 495), but can also be obtained using recombinant DNA technology (see, e.g., U.S. Patent 4,816,567).

As used herein, the term "humanized antibody" refers to an antibody or antibody fragment obtained when all or a part of CDRs of a human immunoglobulin (receptor antibody) is replaced by the CDRs of a non-human antibody (donor antibody), wherein the donor antibody may be a non-human (e.g., mouse, rat or rabbit) antibody having expected specificity, affinity or reactivity. In addition, some amino acid residues in the framework regions (FRs) of the receptor antibody can also be replaced by the amino acid residues of corresponding non-human antibodies or by the amino acid residues of other antibodies to further improve or optimize the performance of the antibody. For more details on humanized antibodies, see, e.g., Jones et al., Nature, 1986; 321: 522-525; Reichmann et al., Nature, 1988; 332: 323-329; Presta, Curr. Op. Struct. Biol., 1992; 2: 593-596; and Clark, Immunol. Today, 2000; 21: 397-402.

As used herein, the term "isolated" refers to obtaining by artificial means from a natural state. If a certain "isolated" substance or component is present in nature, it may be the case that a change occurs in its natural environment, or that it is isolated from the natural environment, or both. For example, a certain non-isolated polynucleotide or polypeptide naturally occurs in a certain living animal, and the same polynucleotide or polypeptide with high purity isolated from such a natural state is referred to as an isolated polynucleotide or polypeptide. The term "isolated" does not exclude the existence of artificial or synthetic substances or other impurities that do not affect the activity of the substance.

As used herein, the term "vector" refers to a nucleic acid vehicle into which a polynucleotide can be inserted. When a vector allows the expression of the protein encoded by the inserted polynucleotide, the vector is referred to as an expression vector. The vector can be introduced into a host cell by transformation, transduction or transfection, such that the genetic substance elements carried by the vector can be expressed in the host cell. Vectors are well known to those skilled in the art, including but not limited to: plasmids; phagemids; cosmids; artificial chromosomes, such as yeast artificial chromosome (YAC), bacterial artificial chromosome (BAC), or P1-derived artificial chromosome (PAC); phages such as lambda phages or M13 phages; and animal viruses. Animal viruses that can be used as vectors include, but are not limited to retroviruses (including lentiviruses), adenoviruses, adeno-associated viruses, herpes viruses (such as herpes simplex virus), poxviruses, baculoviruses, papillomaviruses, and papovaviruses (such as SV40). A vector may comprise a variety of elements that control expression, including, but not limited to, promoter sequences, transcription initiation sequences, enhancer sequences, selection elements, and reporter genes. In addition, the vector may further comprise a replication initiation site. As used herein, the term "host cell" refers to cells to which vectors can be introduced, including, but not limited to, prokaryotic cells such as *E*. *coli* or *bacillus subtilis,* fungal cells such as yeast cells or *aspergillus,* insect cells such as S2 drosophila cells or Sf9, or animal cells such as fibroblasts, CHO cells, GS cells, COS cells, NSO cells, HeLa cells, BHK cells, HEK 293 cells, or human cells.

As used herein, the term "specific binding" refers to a non-random binding reaction between two molecules, such as a reaction between an antibody and an antigen it targets. In some embodiments, an antibody specifically binding to an antigen (or an antibody specific to an antigen) means that the antibody binds to the antigen with an affinity (K_{D}) of less than about 10⁻⁵ M, such as less than about 10⁻⁶ M, 10⁻⁷ M, 10⁻⁸ M, 10⁻⁹ M, or 10⁻¹⁰ M, or less.

As used herein, the term "K_{D}" refers to a dissociation equilibrium constant for a specific antibody-antigen interaction, which is used to describe the binding affinity between the antibody and the antigen. A smaller dissociation equilibrium constant indicates a stronger antibody-antigen binding and a higher affinity between the antibody and the antigen. Generally, antibodies bind to antigens (e.g., PD-1 protein) with a dissociation equilibrium constant (K_{D}) of less than about 10⁻⁵ M, such as less than about 10⁻⁶ M, 10⁻⁷ M, 10⁻⁸ M, 10⁻⁹ M, or 10⁻¹⁰ M, or less. K_{D} can be determined using methods known to those skilled in the art, e.g., using a Fortebio molecular interaction instrument.

As used herein, the terms "monoclonal antibody" and "mAb" have the same meaning and can be used interchangeably; the terms "polyclonal antibody" and "pAb" have the same meaning and can be used interchangeably. Besides, as used herein, amino acids are generally represented by single-letter and three-letter abbreviations known in the art. For example, alanine can be represented by A or Ala.

As used herein, the term "pharmaceutically acceptable carrier and/or excipient" refers to a carrier and/or excipient that is pharmacologically and/or physiologically compatible with the subject and the active ingredient. Such carriers and/or excipients are well known in the art (see, e.g., Remington's Pharmaceutical Sciences, edited by Gennaro AR, 19th Ed., Pennsylvania, Mack Publishing Company, 1995), including but not limited to: pH regulators, surfactants, adjuvants and ionic strength enhancers. For example, the pH regulators include, but are not limited to, phosphate buffer; the surfactants include, but are not limited to, cationic, anionic or non-ionic surfactants, such as Tween-80; the ionic strength enhancers include, but are not limited to, sodium chloride.

As used herein, the term "effective amount" refers to an amount sufficient to obtain or at least partially obtain a desired effect. For example, a prophylactically effective amount against a disease (e.g., a tumor) refers to an amount sufficient to prevent, stop, or delay the onset of the disease (e.g., a tumor); a therapeutically effective amount refers to an amount sufficient to cure or at least partially stop diseases and complications thereof in patients suffering from the disease. It is undoubtedly within the ability of those skilled in the art to determine such an effective amount. For example, the amount effective for therapeutic purpose will depend on the severity of the disease to be treated, the overall state of the patient's own immune system, the general condition of the patient such as age, body weight and gender, the route of administration, and other treatments given concurrently, etc.

As used herein, when referring to the amino acid sequence of lymphocyte-activation gene 3 (LAG3), it includes the full length of LAG3 protein, or the extracellular fragment LAG3 ECD of LAG3, or a fragment comprising LAG3 ECD, and it also includes a fusion protein of the full length of LAG3 protein or a fusion protein of LAG3 ECD, such as a fragment fused to an Fc protein fragment of mouse or human IgG (mFc or hFc). However, those skilled in the art will appreciate that in the amino acid sequence of the LAG3 protein, mutations or variations (including but not limited to, substitutions, deletions, and/or additions) can be naturally produced or artificially introduced without affecting biological functions thereof. Therefore, in the present invention, the term "LAG3 protein" should include all such sequences, including their natural or artificial variants. In addition, when describing a sequence fragment of the LAG3 protein, it also includes the corresponding sequence fragments in their natural or artificial variants.

### Beneficial effects of the present invention

The present invention achieves one or more of the following effects:
(1) the anti-LAG3 antibody of the present invention has superior affinity and specificity; and
(2) the anti-LAG3 antibody of the present invention can effectively block the interaction between LAG3 and MHC-II and specifically relieve the immunosuppression of LAG3 on an organism.

### BRIEF DESCRIPTION OF THE DRAWINGS

FIG. 1 shows the results of an assay for the binding activity of H7L8(hG1WT) to the antigen LAG3-mFc by indirect ELISA.
FIG. 2 shows the results of assays for the binding activity of H7L8(hG4WT), H7L9(hG4WT), and H7L10(hG4WT) to the antigen human LAG3-mFc by ELISA.
FIG. 3 shows the results of assays for the binding activity of H7L8(hG4WT), H7L9(hG4WT), and H7L10(hG4WT) to the antigen LAG3 on 293T-LAG3 cell surface by FACS.
FIG. 4 shows the results of assays for the activity of H7L8(hG4WT), H7L9(hG4WT), and H7L10(hG4WT) in competing with LAG3-mFc for binding to the antigen MHC II on 293T-LAG3 cell membrane surface by competitive flow cytometry.
FIG. 5 shows the results of assays for the biological activity of anti-LAG3 antibodies in promoting IFN-γ secretion by mixed lymphocyte reaction (MLR).
FIG. 6 shows the results of assays for the biological activity of anti-LAG3 antibodies in promoting IL-2 secretion by mixed lymphocyte reaction (MLR).
FIG. 7 shows the results of assays for the biological activity of anti-LAG antibodies in blocking the interaction between LAG-3 and MHC-II.

### DETAILED DESCRIPTION

The embodiments of the present invention will be described in detail below with reference to the examples. Those skilled in the art will appreciate that the following examples are only for illustrating the present invention, and should not be construed as limitations to the scope of the present invention. Examples where the specific technologies or conditions are not specified are performed according to the technologies or conditions described in the publications of the art (e.g., see, Molecular Cloning: A Laboratory Manual, authored by J. Sambrook et al., and translated by Huang Peitang et al., third edition, Science Press) or according to the package insert. Reagents or instruments used are commercially available conventional products if the manufacturers thereof are not specified.

The positive control antibody, Relatlimab, has sequences referenced to the U.S. Patent Publication No. US20160326248A1, wherein the heavy chain amino acid sequence is referenced to SEQ ID NO: 1 of this patent publication and the light chain amino acid sequence is referenced to SEQ ID NO: 2 of this patent publication. Relatlimab is an anti-LAG-3 antibody.
Heavy chain amino acid sequence of Relatlimab: 
QVQLQQWGAGLLKPSETLSLTCAVYGGSFSDYYWNWIRQPPGKGLEWIGEINHR
GSTNSNPSLKSRVTLSLDTSKNQFSLKLRSVTAADTAVYYCAFGYSDYEYNWFDPW
GQGTLVTVSSASTKGPSVFPLAPCSRSTSESTAALGCLVKDYFPEPVTVSWNSGALT
SGVHTFPAVLQSSGLYSLSSVVTVPSSSLGTKTYTCNVDHKPSNTKVDKRVESKYGP
PCPPCPAPEFLGGPSVFLFPPKPKDTLMISRTPEVTCVVVDVSQEDPEVQFNWYVD
GVEVHNAKTKPREEQFNSTYRVVSVLTVLHQDWLNGKEYKCKVSNKGLPSSIEKTI
SKAKGQPREPQVYTLPPSQEEMTKNQVSLTCLVKGFYPSDIAVEWESNGQPENNY
KTTPPVLDSDGSFFLYSRLTVDKSRWQEGNVFSCSVMHEALHNHYTQKSLSLSLGK
(SEQ ID NO: 23)
Light chain amino acid sequence of Relatlimab:

EIVLTQSPATLSLSPGERATLSCRASQSISSYLAWYQQKPGQAPRLLIYDASNRATGI
PARFSGSGSGTDFTLTISSLEPEDFAVYYCQQRSNWPLTFGQGTNLEIKRTVAAPSV
FIFPPSDEQLKSGTASVVCLLNNFYPREAKVQWKVDNALQSGNSQESVTEQDSKDS
TYSLSSTLTLSKADYEKHKVYACEVTHQGLSSPVTKSFNRGEC (SEQ ID NO: 24)

The control antibody 14C12H1L1(hG1TM) was an anti-PD-1 antibody constructed by Akeso Biopharma Inc. with a Batch No. B105Y2080601.
Heavy chain amino acid sequence of 14C12H1L1(hG1TM): 
EVQLVESGGGLVQPGGSLRLSCAASGFAFSSYDMSWVRQAPGKGLDWVATISGGG
RYTYYPDSVKGRFTISRDNSKNNLYLQMNSLRAEDTALYYCANRYGEAWFAYWG
QGTLVTVSSASTKGPSVFPLAPSSKSTSGGTAALGCLVKDYFPEPVTVSWNSGALTS
GVHTFPAVLQSSGLYSLSSVVTVPSSSLGTQTYICNVNHKPSNTKVDKKVEPKSCDK
THTCPPCPAPEAAGAPSVFLFPPKPKDTLMISRTPEVTCVVVDVSHEDPEVKFNWY
VDGVEVHNAKTKPREEQYNSTYRVVSVLTVLHQDWLNGKEYKCKVSNKALPAPIE
KTISKAKGQPREPQVYTLPPSRDELTKNQVSLTCLVKGFYPSDIAVEWESNGQPEN
 
NYKTTPPVLDSDGSFFLYSKLTVDKSRWQQGNVFSCSVMHEALHNHYTQKSLSLSP
GK (SEQ ID NO: 21)
Light chain amino acid sequence of 14C12H1L1(hG1TM):

DIQMTQSPSSMSASVGDRVTFTCRASQDINTYLSWFQQKPGKSPKTLIYRANRLVS
GVPSRFSGSGSGQDYTLTISSLQPEDMATYYCLQYDEFPLTFGAGTKLELKRTVAA
PSVFIFPPSDEQLKSGTASVVCLLNNFYPREAKVQWKVDNALQSGNSQESVTEQDS
KDSTYSLSSTLTLSKADYEKHKVYACEVTHQGLSSPVTKSFNRGEC (SEQ ID NO: 22)

The cell line 293T-LAG3 was constructed by Akeso Biopharma Inc. The cell line 293T-LAG3 was produced by viral infection of HEK293T cells using 3rd Generation Lentiviral Systems (see, e.g., A Third Generation Lentivirus Vector with a Conditional Packaging System. Dull T, Zufferey R, Kelly M, Mandel RJ, Nguyen M, Trono D, and Naldini L., J Virol., 1998. 72(11): 8463-8471), wherein the lentivirus expression vector used was plenti6.3/V5-huLAG3FL-BSD (LAG3, Genebank ID: NM 002277.4; vector plenti6.3/V5-BSD, purchased from Invitrogen, Cat. No. K5315-20).

The cell line Raji-PDL1 was constructed by Akeso Biopharma Inc. The cell line Raji-PDL1 was produced by viral infection of Raji cells using 3rd Generation Lentiviral Systems (see, e.g., A Third Generation Lentivirus Vector with a Conditional Packaging System. Dull T, Zufferey R, Kelly M, Mandel RJ, Nguyen M, Trono D, and Naldini L., J Virol., 1998. 72(11): 8463-8471), wherein the lentivirus expression vector used was plenti6.3/V5-PDLl (PDL1, Genebank ID: NP_054862.1; vector plenti6.3/V5, purchased from Invitrogen, Cat. No. K5315-20).

The cell line Jurkat-NFAT-PD1-LAG3 was constructed by Akeso Biopharma Inc. The cell line Jurkat-NFAT-PD1-LAG3 was prepared by viral infection of PD-1 effector cells (CPM, manufacturer: Promega, Cat. No. J112A) using 3rd Generation Lentiviral Systems (see, e.g., A Third Generation Lentivirus Vector with a Conditional Packaging System. Dull T, Zufferey R, Kelly M, Mandel RJ, Nguyen M, Trono D, and Naldini L., J Virol., 1998. 72(11): 8463-8471), wherein the lentivirus expression vector used was pCDH-huLAG3FL-RFP-NEO (LAG3, Genebank ID: NM 002277.4; vector pCDH-CMV-MCS-EF1-RFP+Neo, purchased from Youbio, Cat. No. VT9005).

### Preparation Example 1: Design and Preparation of Anti-LAG3 Antibodies

### 1. Design of antibodies

The inventors creatively designed a series of antibody sequences based on the known LAG3 protein sequence (NCBI Reference Sequence: NP_002277.4) and the three-dimensional crystal structure thereof, etc. Through extensive screening and testing, humanized monoclonal antibodies specifically binding to LAG3 were finally obtained, named H7L8, H7L9 and H7L10, respectively. The amino acid sequences of the heavy and light chain variable regions of the monoclonal antibodies and the encoding sequences thereof are as follows.
Nucleotide sequence of the heavy chain variable region H7v of H7L8 (360 bp): 
CAGGTGCAGCTGCAGCAGTGGGGAGCTGGACTGCTGAAACCTAGCGAGACACT
GAGCCTGACCTGTGCTGTGTACGGCGGATCTATCAGCGATTACTACTGGAACT
GGATCAGGCAGCCCCCTGGAAAGGGACTGGAATGGATCGGAGAGATCAACCAC
AGGGGCACCACCAACTCCAATCCCTCTCTGAAGAGCAGGGTGACACTGAGCCT
CGACACAAGCAAGAATCAGTTCAGCCTGAAGCTGAGGTCCGTGACCGCTGCTG
ATACAGCTGTGTACTACTGTGCCTTCGGCTACAGCGATTACGAGTACGATTGGT
TCGACCCTTGGGGCCAGGGAACACTGGTTACAGTGAGCTCC (SEQ ID NO: 1)
Amino acid sequence of the heavy chain variable region H7v of H7L8 (120 aa): 
QVQLQQWGAGLLKPSETLSLTCAVYGGSISDYYWNWIRQPPGKGLEWIGEINHRG
TTNSNPSLKSRVTLSLDTSKNQFSLKLRSVTAADTAVYYCAFGYSDYEYDWFDPWG
QGTLVTVSS (SEQ ID NO: 2)
Nucleotide sequence of the light chain variable region L8v of H7L8 (321 bp): 
GAGATCGTTCTGACCCAGAGCCCAGCTACACTGAGCCTGTCTCCTGGAGAGAG
GGCTACACTGTCCTGCAGAGCTAGCCAGACCATCAGCAGCTACCTGGCTTGGT
ACCAGCAGAAGCCTGGCCAAGCTCCAAGGCTGCTGATCTACGACGCCTCTAAT
AGGGCCACCGGCATCCCTGCTAGATTCTCTGGAAGCGGCAGCGGAACCGACTT
TACACTGACAATCAGCTCCCTGGAGCCCGAGGATTTCGCTGTTTACTACTGTCA
 
GCAGCGCAGCAACTGGCCCATCACATTCGGACAGGGCACAAATCTGGAGATCA
AG (SEQ ID NO: 3)
Amino acid sequence of the light chain variable region L8v of H7L8 (107 aa):

EIVLTQSPATLSLSPGERATLSCRASQTISSYLAWYQQKPGQAPRLLIYDASNRATGI
PARFSGSGSGTDFTLTISSLEPEDFAVYYCQQRSNWPITFGQGTNLEIK (SEQID NO:
4)

The nucleotide sequence of the heavy chain variable region H7v of H7L9 is identical to the nucleotide sequence of the heavy chain variable region H7v of H7L8, as set forth in SEQ ID NO: 1.

The amino acid sequence of the heavy chain variable region H7v of H7L9 is identical to the amino acid sequence of the heavy chain variable region H7v of H7L8, as set forth in SEQ ID NO: 2.
Nucleotide sequence of the light chain variable region L9v of H7L9 (321 bp): 
GAGATCGTTCTGACCCAGAGCCCAGCTACACTGAGCCTGTCTCCTGGAGAGAG
GGCTACACTGTCCTGCAGAGCTAGCCAGACCATCAGCAGCTACCTGGCTTGGT
ACCAGCAGAAGCCTGGCCAAGCTCCAAGGCTGCTGATCTACGACGGCTCTAAT
AGGGCCACCGGCATCCCTGCTAGATTCTCTGGAAGCGGCAGCGGAACCGACTT
TACACTGACAATCAGCTCCCTGGAGCCCGAGGATTTCGCTGTTTACTACTGTCA
GCAGCGCAGCAACTGGCCCCTCACATTCGGACAGGGCACAAATCTGGAGATCA
AG (SEQ ID NO: 5)
Amino acid sequence of the light chain variable region L9v of H7L9 (107 bp):

EIVLTQSPATLSLSPGERATLSCRASQTISSYLAWYQQKPGQAPRLLIYDGSNRATGI
PARFSGSGSGTDFTLTISSLEPEDFAVYYCQQRSNWPLTFGQGTNLEIK (SEQ ID NO:
6)

The nucleotide sequence of the heavy chain variable region H7v of H7L10 is identical to the nucleotide sequence of the heavy chain variable region H7v of H7L8, as set forth in SEQ ID NO: 1.

The amino acid sequence of the heavy chain variable region H7v of H7L10 is identical to the amino acid sequence of the heavy chain variable region H7v of H7L8, as set forth in SEQ ID NO: 2.
Nucleotide sequence of the light chain variable region L10v of H7L10 (321 bp): 
GAGATCGTTCTGACCCAGAGCCCAGCTACACTGAGCCTGTCTCCTGGAGAGAG
GGCTACACTGTCCTGCAGAGCTAGCCAGTCCATCAGCAGCTACCTGGCTTGGT
ACCAGCAGAAGCCTGGCCAAGCTCCAAGGCTGCTGATCTACGACGGCTCTAAT
AGGGCCACCGGCATCCCTGCTAGATTCTCTGGAAGCGGCAGCGGAACCGACTT
TACACTGACAATCAGCTCCCTGGAGCCCGAGGATTTCGCTGTTTACTACTGTCA
GCAGCGCAGCAACTGGCCCATCACATTCGGACAGGGCACAAATCTGGAGATCA
AG (SEQ ID NO: 7)
Amino acid sequence of the light chain variable region L10v of H7L10 (107 bp):

EIVLTQSPATLSLSPGERATLSCRASQSISSYLAWYQQKPGQAPRLLIYDGSNRATGI
PARFSGSGSGTDFTLTISSLEPEDFAVYYCQQRSNWPITFGQGTNLEIK (SEQ ID NO:
8)

The amino acid sequences of the CDRs of the antibody H7L8 are as follows (according to the IMGT numbering system):
HCDR1: GGSISDYY (SEQ ID NO: 9);
HCDR2: INHRGTT (SEQ ID NO: 10);
HCDR3: AFGYSDYEYDWFDP (SEQ ID NO: 11);
LCDR1: QTISSY (SEQ ID NO: 12);
LCDR2: DAS (SEQ ID NO: 13); and
LCDR3: QQRSNWPIT (SEQ ID NO: 14).

The amino acid sequences of the CDRs of the antibody H7L9 are as follows (according to the IMGT numbering system):
HCDR1: GGSISDYY (SEQ ID NO: 9);
HCDR2: INHRGTT (SEQ ID NO: 10);
HCDR3: AFGYSDYEYDWFDP (SEQ ID NO: 11);
LCDR1: QTISSY (SEQ ID NO: 12);
LCDR2: DGS (SEQ ID NO: 15); and
LCDR3: QQRSNWPLT (SEQ ID NO: 16).

The amino acid sequences of the CDRs of the antibody H7L10 are as follows (according to the IMGT numbering system):
HCDR1: GGSISDYY (SEQ ID NO: 9);
HCDR2: INHRGTT (SEQ ID NO: 10);
HCDR3: AFGYSDYEYDWFDP (SEQ ID NO: 11);
LCDR1: QSISSY (SEQ ID NO: 17);
LCDR2: DGS (SEQ ID NO: 15); and
LCDR3: QQRSNWPIT (SEQ ID NO: 14).

### 2. Expression and purification of humanized antibody H7L8(hG1WT)

The heavy chain cDNA sequence (the encoding sequence of the variable region was set forth in SEQ ID NO: 1; the constant region was Ig gamma-1 chain C region) and the light chain cDNA sequence (the encoding sequence of the variable region was set forth in SEQ ID NO: 3; the constant region was human Ig kappa chain C region) of H7L8(hG1WT) were separately cloned into pUC57simple vectors (supplied by GenScript), and plasmids pUC57simple-H7 and pUC57simple-L8 were obtained, respectively. The plasmids pUC57simple-H7 and pUC57simple-L8 were each digested (HindIII&EcoRI). The heavy and light chains isolated by electrophoresis were separately subcloned into pcDNA3.1 vectors, and recombinant plasmids were extracted to co-transfect 293F cells. After 7 days of cell culture, the culture medium was separated by high-speed centrifugation, and the supernatant was concentrated and loaded onto a HiTrap MabSelect SuRe column. The protein was eluted in one step with an elution buffer. The target sample was isolated, and the buffer was exchanged into PBS.
Amino acid sequence of the heavy chain constant region of H7L8(hG1WT) 





 (SEQ ID
NO: 18)
Amino acid sequence of the light chain constant region of H7L8(hG1WT)


 (SEQ ID
NO: 19)

### 3. Expression and purification of humanized antibodies H7L8(hG4WT), H7L9(hG4WT) and H7L10(hG4WT)

The heavy chain cDNA sequences (the encoding sequences of the variable regions were set forth in SEQ ID NO: 1; the constant regions were Ig gamma-4 chain C regions) of H7L8(hG4WT), H7L9(hG4WT) and H7L10(hG4WT), the light chain cDNA sequence (the encoding sequence of the variable region was set forth in SEQ ID NO: 3; the constant region was human Ig kappa chain C region) of H7L8(hG4WT), the light chain cDNA sequence (the encoding sequence of the variable region was set forth in SEQ ID NO: 5; the constant region was human Ig kappa chain C region) of H7L9(hG4WT), and the light chain cDNA sequence (the encoding sequence of the variable region was set forth in SEQ ID NO: 7; the constant region was human Ig kappa chain C region) of H7L10(hG4WT) were separately cloned into pUC57simple vectors (supplied by GenScript), and plasmids pUC57simple-H7, pUC57simple-L8, pUC57simple-L9 and pUC57simple-L10 were obtained, respectively. The plasmids pUC57simple-H7, pUC57simple-L8, pUC57simple-L9 and pUC57simple-L10 were each digested (HindIII&EcoRI). The heavy and light chains isolated by electrophoresis were separately subcloned into pcDNA3.1 vectors, and recombinant plasmids were extracted to co-transfect 293F cells. After 7 days of cell culture, the culture medium was separated by high-speed centrifugation, and the supernatant was concentrated and loaded onto a HiTrap MabSelect SuRe column. The protein was eluted in one step with an elution buffer. The target sample was isolated, and the buffer was exchanged into PBS.
Amino acid sequence of the heavy chain constant region of H7L8(hG4WT), H7L9(hG4WT), or H7L10(hG4WT): 
ASTKGPSVFPLAPCSRSTSESTAALGCLVKDYFPEPVTVSWNSGALTSGVHTFPAVL
QSSGLYSLSSVVTVPSSSLGTKTYTCNVDHKPSNTKVDKRVESKYGPPCPPCPAPEF
LGGPSVFLFPPKPKDTLMISRTPEVTCVVVDVSQEDPEVQFNWYVDGVEVHNAKT
KPREEQFNSTYRVVSVLTVLHQDWLNGKEYKCKVSNKGLPSSIEKTISKAKGQPRE
PQVYTLPPSQEEMTKNQVSLTCLVKGFYPSDIAVEWESNGQPENNYKTTPPVLDSD
GSFFLYSRLTVDKSRWQEGNVFSCSVMHEALHNHYTQKSLSLSLGK (SEQ ID NO:
20)
Amino acid sequence of the light chain constant region of H7L8(hG4WT), H7L9(hG4WT), or H7L10(hG4WT):


 (SEQ ID
NO: 19)

### Preparation Example 2: Preparation of Human Anti-Hen Egg Lysozyme Antibody

The sequence of the human anti-hen egg lysozyme IgG (anti-HEL, or human IgG, abbreviated as hIgG) antibody was derived from the variable region sequence of the Fab F10.6.6 sequence in the study reported by Acierno et al., entitled "Affinity maturation increases the stability and plasticity of the Fv domain of anti-protein antibodies" (Acierno et al., J Mol Biol., 2007; 374(1): 130-46). The preparation method was as follows:
Nanjing Genscript Biology was entrusted to carry out codon optimization of amino acids and gene synthesis on heavy and light chain (complete sequence or variable region) genes of the human IgG antibody, and by referring to the standard technologies introduced in the "Guide to Molecular Cloning Experiments (Third Edition)" and using standard molecular cloning techniques such as PCR, enzyme digestion, DNA gel extraction, ligation transformation, colony PCR or enzyme digestion identification, the heavy and light chain genes were subcloned into the antibody heavy chain expression vector and antibody light chain expression vector of the mammalian expression system, respectively. The heavy and light chain genes of the recombinant expression vectors were further sequenced and analyzed. After the sequences were verified to be correct, a medium or large amount of endotoxin-free expression plasmids were prepared, and the heavy and light chain expression plasmids were transiently co-transfected into HEK293 cells for recombinant antibody expression. After 7 days of culture, the cell culture medium was collected and subjected to affinity purification using an rProtein A column (GE), and the quality of the resulting antibody sample was determined using SDS-PAGE and SEC-HPLC standard analysis techniques.

### Experimental Example 1: Assays for Binding Activity of Anti-LAG3 Antibodies to Antigen by ELISA

An ELISA plate was coated with human LAG3-mFc (constructed by Akeso Biopharma Inc., Batch No. 20200417) at 0.5 µg/mL and incubated at 4 °C overnight. Then the ELISA plate coated with the antigen was washed once with PBST and then blocked with a PBS solution containing 1% BSA as a blocking solution at 37 °C for 2 h. After blocking, the ELISA plate was washed 3 times with PBST. The antibodies serially diluted with PBST solution (the dilution gradients for the antibody are shown in Table 1) were added. The ELISA plate containing the test antibodies was incubated at 37 °C for 30 min and then washed 3 times with PBST. After washing, a working solution of an HRP-labeled goat anti-human IgG FC (H+L) (Jackson, Cat. No. 109-035-098) secondary antibody diluted at a ratio of 1:5000 was added, and then the plate was incubated at 37 °C for 30 min. After incubation, the plate was washed 4 times with PBST, TMB (Neogen, 308177) was added for chromogenesis in the dark for 5 min, and then a stop solution was added to terminate the chromogenic reaction. The ELISA plate was put into an ELISA plate reader immediately, and the OD value of each well in the ELISA plate was read at 450 nm. The data were analyzed and processed by SoftMax Pro 6.2.1.

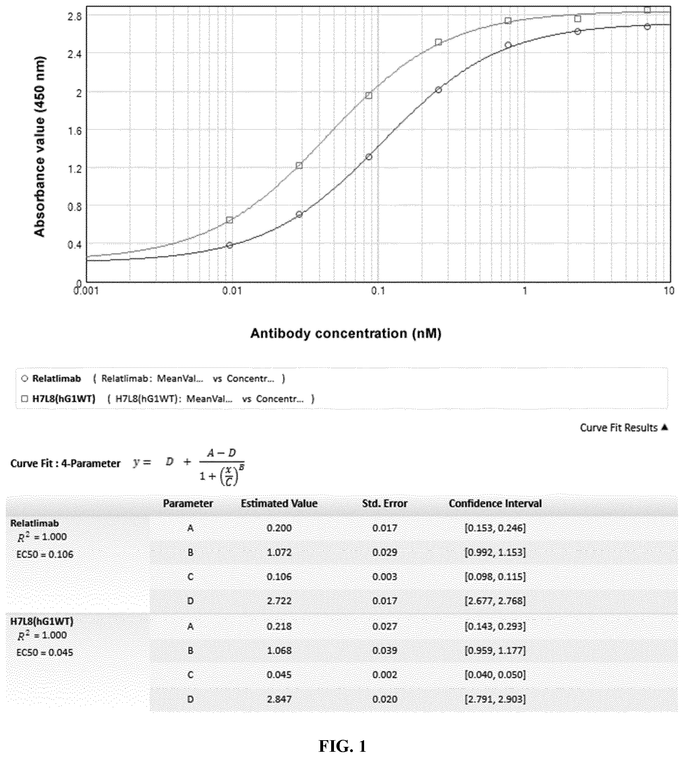

The assay results are shown in Table 1 and FIG. 1.

**Table 1: Results of assays for the binding of Relatlimab and H7L8(hG1WT) to LAG3-mFc by ELISA**

| Antibody concentration | Antigen-antibody binding OD (450 nm) value | | | |
|---|---|---|---|---|
| (nM) | Relatlimab | | H7L8(hG1WT) | |
| 7.000 | 2.703 | 2.660 | 2.852 | 2.861 |
| 2.333 | 2.620 | 2.640 | 2.746 | 2.787 |
| 0.778 | 2.478 | 2.486 | 2.687 | 2.788 |
| 0.259 | 2.006 | 2.025 | 2.466 | 2.569 |
| 0.086 | 1.294 | 1.322 | 1.895 | 2.019 |
| 0.029 | 0.703 | 0.719 | 1.176 | 1.269 |
| 0.010 | 0.394 | 0.376 | 0.620 | 0.681 |
| 0 | 0.194 | 0.187 | 0.207 | 0.221 |
| EC₅₀(nM) | 0.106 | | 0.045 | |

As can be seen from FIG. 1, Relatlimab and H7L8(hG1WT) could effectively bind to the antigen human LAG3-mFc in a dose-dependent manner. The absorbance intensity for each dose is shown in Table 1. By quantitative analysis of the absorbance of the bound antibodies, the binding efficiency EC₅₀ values of the antibodies Relatlimab (as a positive control) and H7L8(hG1WT) obtained by curve fitting calculation were 0.106 nM and 0.045 nM, respectively.

The above experimental results show that under the same experimental conditions, H7L8(hG1WT) had the activity of effectively binding to human LAG3-mFc, and the binding activity of H7L8(hG1WT) to human LAG3-mFc was stronger than that of the positive drug Relatlimab for the same target.

### Example 2: Assays for Binding Activity of Anti-LAG3 Antibodies to Antigen by ELISA

An ELISA plate was coated with human LAG3-mFc at 2 µg/mL and incubated at 4 °C overnight. Then the ELISA plate coated with the antigen was washed once with PBST and then blocked with a PBS solution containing 1% BSA as a blocking solution at 37 °C for 2 h.

After blocking, the ELISA plate was washed 3 times with PBST. The antibodies serially diluted with PBST solution (the dilution gradients for the antibody are shown in Table 1) were added. The ELISA plate containing the test antibodies was incubated at 37 °C for 30 min and then washed 3 times with PBST. After washing, a working solution of an HRP-labeled goat anti-human IgG (H+L) (Jackson, Cat. No. 109-035-088) secondary antibody diluted at a ratio of 1:5000 was added, and then the plate was incubated at 37 °C for 30 min. After incubation, the plate was washed 4 times with PBST, TMB (Neogen, 308177) was added for chromogenesis in the dark for 5 min, and then a stop solution was added to terminate the chromogenic reaction. The ELISA plate was put into an ELISA plate reader immediately, and the OD value of each well in the ELISA plate was read at 450 nm. The data were analyzed and processed by SoftMax Pro 6.2.1.

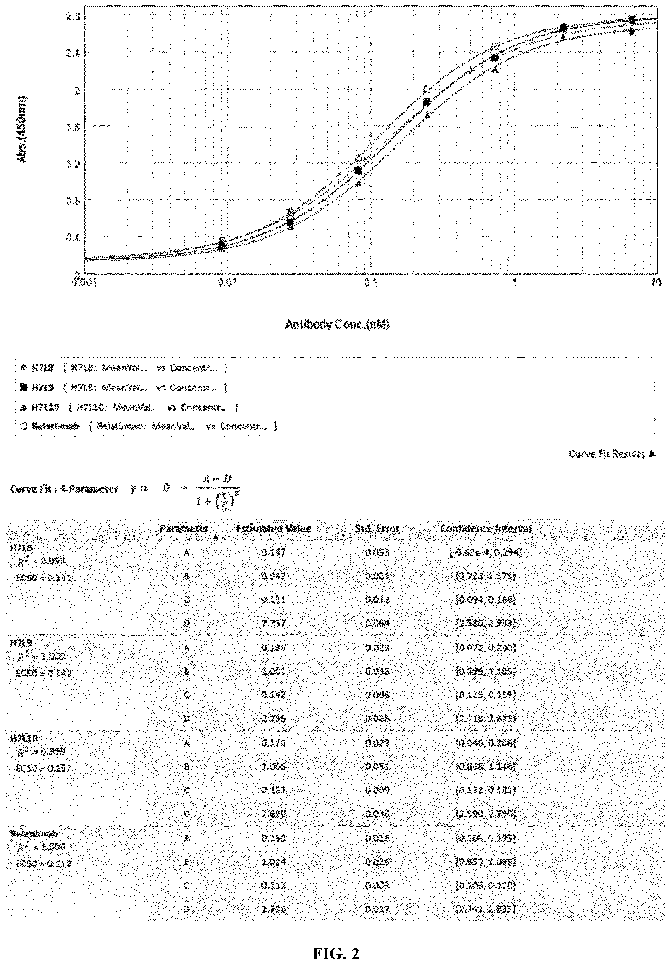

The assay results are shown in Table 2 and FIG. 2.

**Table 2: Results of assays for the binding of H7L8(hG4WT), H7L9(hG4WT), and H7L10(hG4WT) to antigen human LAG3-mFc by ELISA**

| | Human LAG3-mFc, 2 µg/mL, 50 µL/well | | | | | | | |
|---|---|---|---|---|---|---|---|---|
| Antibody dilution (µg/mL) | H7L8 (hG4WT) | | H7L9 (hG4WT) | | H7L10 (hG4WT) | | Relatlimab | |
| 1 | 2.659 | 2.600 | 2.735 | 2.745 | 2.610 | 2.626 | 2.748 | 2.747 |
| 0.3 | 2.724 | 2.596 | 2.668 | 2.637 | 2.574 | 2.548 | 2.664 | 2.674 |
| 0.1 | 2.397 | 2.296 | 2.326 | 2.334 | 2.201 | 2.211 | 2.426 | 2.480 |
| 0.03 | 1.878 | 1.765 | 1.853 | 1.852 | 1.716 | 1.735 | 1.936 | 2.048 |
| 0.01 | 1.210 | 1.050 | 1.106 | 1.102 | 0.968 | 1.005 | 1.194 | 1.309 |
| 0.003 | 0.581 | 0.775 | 0.534 | 0.577 | 0.487 | 0.523 | 0.571 | 0.727 |
| 0.001 | 0.313 | 0.379 | 0.304 | 0.307 | 0.270 | 0.275 | 0.348 | 0.381 |
| 0 | 0.123 | 0.130 | 0.141 | 0.130 | 0.123 | 0.120 | 0.135 | 0.138 |

| Secondary antibody | Goat Anti Human IgG(H+L), HRP(1:5000) | | | | | | | |
|---|---|---|---|---|---|---|---|---|
| EC₅₀ (nM) | 0.131 | | 0.142 | | 0.157 | | 0.112 | |

The results show that the antibodies H7L8(hG4WT), H7L9(hG4WT) and H7L10(hG4WT) could effectively bind to the antigen human LAG3-mFc in a dose-dependent manner, and had binding activity comparable to that of the positive control antibody Relatlimab.

### Example 3: Assays for Binding Activity of Anti-LAG3 antibodies to Antigen LAG3 on Cell Surface by Flow Cytometry

### 1. Construction of 293T host cells expressing antigen LAG3

The procedures were as follows:
Construction of 293T host cells expressing antigen LAG3: A vector pLenti6.3/V5-huLAG3FL-BSD containing LAG3 (the vector pLenti6.3 was purchased from Invitrogen) was transfected into 293T cells according to the instructions of a lipofectamin transfection kit (purchased from Invitrogen), and a clone group 293T-LAG3 stably expressing LAG3 was obtained by screening.

### 2. Binding of antibodies to antigen on 293T-LAG3 cell surface

Antibody labeling and flow cytometer detection: The 293T-LAG3 host cells expressing antigen LAG3 obtained in the previous step were digested with conventional pancreatin, and the number of cells in each collection tube was made to be 3×10⁵. LAG3 antibody dilutions prepared using 1% PBSA (PBS containing 1% BSA) at final concentrations of 0.0123 nM, 0.123 nM, 1.23 nM, 3.7 nM, 11.1 nM, 33.3 nM, 100 nM, and 300 nM, respectively, were each incubated with the 293T cells expressing LAG3 on ice for 1 h. After centrifugation and washing several times with 1% PBSA, 100 µL of FITC goat anti-human IgG (purchased from Jackson, Cat. No. 109-095-098) (diluted at a 1:500 ratio) was added into each tube, and the mixture was incubated on ice in the dark for 40 min. After washing once with 1% PBSA, 200 µL of 1% PBSA was added to resuspend the cells. Fluorescence signals were detected with FITC channel on a flow cytometer.

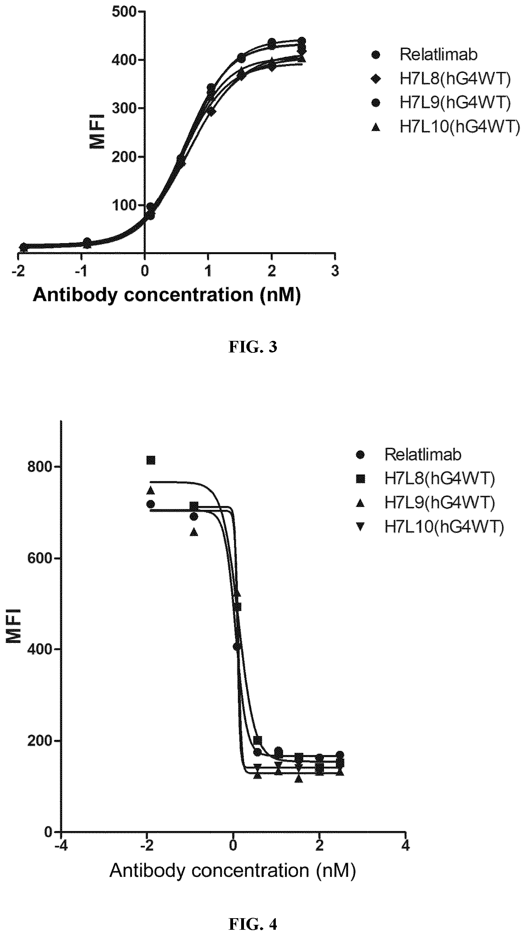

The results of the binding of humanized anti-LAG3 antibodies to 293T-LAG3 cells are shown in FIG. 3. The binding efficiency EC₅₀ values of the anti-LAG3 antibodies to the antigen on 293T-LAG3 cell surface are shown in Table 3.

**Table 3: Results of assays for the binding activity of anti-LAG3 antibodies to antigen on 293T-LAG3 cell surface by flow cytometer**

| | EC₅₀ (nM) |
|---|---|
| Relatlimab | 4.289 |
| H7L8(hG4WT) | 4.929 |
| H7L9(hG4WT) | 4.809 |
| H7L10(hG4WT) | 4.168 |

As can be seen from FIG. 3, the anti-LAG3 antibodies could effectively bind to the target LAG3 protein on the surface of the 293T-LAG3 host cells, and the binding activity of the anti-LAG3 antibodies H7L8(hG4WT), H7L9(hG4WT) and H7L10(hG4WT) to the antigen on 293T-LAG3 cell surface was comparable to that of the positive control antibody Relatlimab.

### Example 4: Assays for Competitive Binding Activity of Anti-LAG3 Antibodies in Competing with LAG3-mFc for Binding to Antigen MHC II on Cell Membrane Surface by Competitive Flow cytometry

Raji cells (medium: 1640 + 10% FBS) (Cell Resource Center, Shanghai Institutes for Biological Sciences, Chinese Academy of Sciences, Cat. No. TCHu 44) were added into EP tubes at 300,000 cells per sample. 1000 µL of 1% PBSA (PBS containing 1% BSA) was added. The mixture was centrifuged at 600× g for 5 min, and the supernatant was discarded. According to the experimental design, 100 µL of hIgG1 (constructed by Akeso Biopharma Inc., Batch No. 20190410) at a final concentration of 300 nM was added into each tube, and the mixture was incubated on ice for 1 h; 200 µL of 1% PBSA was added to the Raji cells after incubation, and the mixture was centrifuged at 600× g for 5 min, followed by removal of the supernatant. Meanwhile, according to the experimental design, correspondingly diluted antibodies (at concentrations of 300 nM, 100 nM, 33.3 nM, 11.1 nM, 3.7 nM, 1.23 nM, 0.123 nM, and 0.0123 nM, respectively) were added into additional clean EP tubes at 60 µL/tube, and a Blank group (PBSA + cells) was designed; then 60 µL of LAG3-mFc (constructed by Akeso Biopharma Inc., Batch No. 20190508) (at a final concentration of 3 nM) was added to each corresponding antibody tube. The mixture was mixed well and pre-incubated on ice for 30 min. 100 µL of the pre-incubated mixture of antibody and protein was added to the sample. The mixture was mixed well and incubated on ice in the dark for 1 h; 200 µL of 1% PBSA was added, and the mixture was centrifuged at 600× g for 5 min, followed by removal of the supernatant, and then washed twice; 100 µL of an APC anti mouse antibody (purchased from Biolegend, Cat. No. 405308) (diluted at a 1:400 ratio) was added, and the mixture was mixed well and incubated on ice in the dark for 40 min; 200 µL of 1% PBSA was added, and the mixture was centrifuged at 600× g for 5 min, followed by removal of the supernatant; 200 µL of Washing Buffer was added into each tube to resuspend the cells, and then the suspension was transferred to a sample loading tube for testing on a flow cytometer.

The results are shown in FIG. 4 and Table 4. By fluorescence quantitative analysis and curve fitting, the competitive binding EC₅₀ values of the antibodies Relalimab, H7L8(hG4WT), H7L9(hG4WT) and H7L10(hG4WT) were calculated to be 1.153 nM, 1.342 nM, 1.317 nM, and 1.267 nM, respectively.

**Table 4: Analysis results of fluorescence intensities of Relalimab, H7L8(hG4WT), H7L9(hG4WT), and H7L10(hG4WT) in competing for binding to antigen on Raji cell surface determined by FACS**

| | EC₅₀ (nM) |
|---|---|
| Relatlimab | 1.153 |
| H7L8(hG4WT) | 1.342 |
| H7L9(hG4WT) | 1.317 |
| H7L10(hG4WT) | 1.267 |

The results show that the antibodies H7L8(hG4WT), H7L9(hG4WT) and H7L10(hG4WT) could effectively block the binding of LAG-3 to MHC II on the surface of Raji host cells in a dose-dependent manner, and had the activity comparable to that of the positive control antibody Relatlimab.

### Experimental Example 5: Assays for Biological Activity of Anti-LAG3 Antibodies in Promoting IFN-γ and IL-2 Secretion by Mixed Lymphocyte Reaction (MLR)

### 1. Assays for biological activity of anti-LAG3 antibodies in promoting IFN-γ secretion in Raji-PDL1 mixed lymphocyte reaction system

Raji-PDL1 cells were conventionally subcultured. PBMCs were thawed, cultured in 10 mL of a 1640 complete medium, and stimulated with SEB (Staphylococcal enterotoxin B) (Dianotech, Cat. No.: S010201) at 0.5 µg/mL for two days. The Raji-PDL1 cells were treated with MMC (Stressmarq, Cat. No. SIH-246-10MG) at 25 µg/mL, and incubated at 37 °C in a 5% CO₂ incubator for 1 h; the PBMCs stimulated with SEB for 2 days and the Raji-PDL1 cells treated with MMC for 1 h were collected, washed twice with PBS, then resuspended in a complete medium (i.e., RPMI 1640 + 10% FBS), and counted. The PBMCs and Raji-PDL1 cells were separately added to a U-shaped 96-well plate (Corning, Model No. 3799) at 10×10⁴ cells/well and co-cultured. According to the experimental design, the antibodies (the final concentrations of each antibody were 300 nM, 30 nM, and 3 nM when used alone or in combination) were added and co-cultured with the cells in an incubator for 3 days; after 3 days, the cells were centrifuged at 1200 rpm for 5 min, and the cell culture supernatant was collected and assayed for IFN-γ by ELISA.

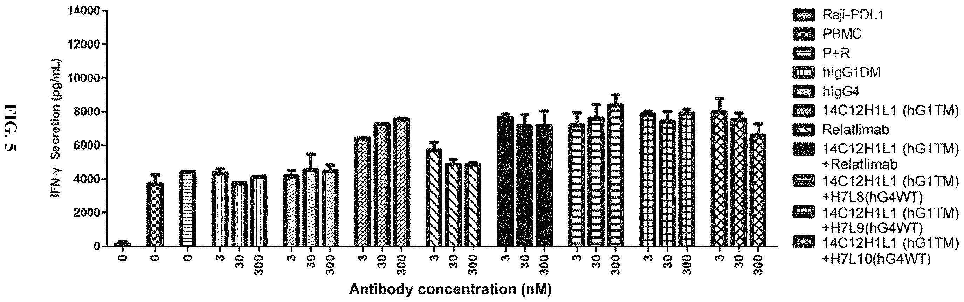

As shown in FIG. 5, the mixed culture of human PBMCs and Raji-PDL1 cells promoted the secretion of IFN-γ in PBMCs, and the addition of the antibodies to the mixed culture system could significantly induce the further secretion of IFN-γ in PBMCs. In terms of the level of activity in promoting IFN-γ secretion, the anti-LAG3 antibodies H7L8(hG4WT), H7L9(hG4WT) and H7L10(hG4WT) each in combination with 14C12H1L1(hG1TM), and the positive control antibody Relatlimab in combination with 14C12H1L1(hG1TM) could promote IFN-γ secretion, with comparable activities.

### 2. Assays for biological activity of anti-LAG antibodies in promoting IL-2 secretion in Raji-PDL1 mixed lymphocyte reaction system

Raji-PDL1 cells were conventionally subcultured. PBMCs were thawed, cultured in 10 mL of a 1640 complete medium, and stimulated with SEB (Staphylococcal enterotoxin B, purchased from Dianotech, Cat. No. S010201) at 0.5 µg/mL for two days. The Raji-PDL1 cells were treated with MMC (Stressmarq, Cat. No. SIH-246-10MG) at 25 µg/mL and incubated at 37 °C in a 5% CO₂ incubator for 1 h. The PBMCs stimulated with SEB for 2 days and the Raji-PDL1 cells treated with MMC for 1 h were collected, washed twice with PBS, then resuspended in a complete medium (i.e., RPMI 1640 + 10% FBS), and counted. The PBMCs and Raji-PDL1 cells were separately added to a U-shaped 96-well plate (Corning, Model No. 3799) at 10×10⁴ cells/well and co-cultured. According to the experimental design, the antibodies (the final concentrations of each antibody were 300 nM, 30 nM, and 3 nM when used alone or in combination) were added and co-cultured with the cells for 3 days; after 3 days, the cells were centrifuged at 1200 rpm for 5 min, and the cell culture supernatant was collected and assayed for IL-2 by ELISA.

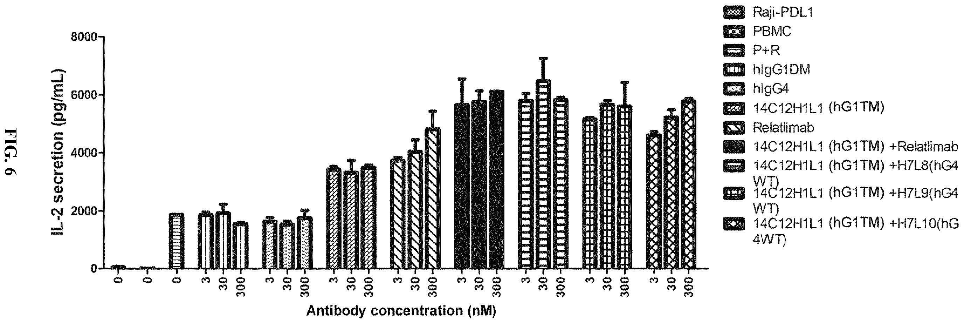

As shown in FIG. 6, the mixed culture of human PBMCs (from healthy donors) and Raji-PDL1 cells promoted the secretion of IL-2 in PBMCs to some extent, and the addition of the antibodies to the mixed culture system could significantly induce the further secretion of IL-2 in PBMCs, exhibiting a significant dose-dependent relationship. In terms of the level of activity in promoting IL-2 secretion, the anti-LAG3 antibodies H7L8(hG4WT), H7L9(hG4WT) and H7L10(hG4WT) each in combination with 14C12H1L1(hG1TM), and the positive control antibody Relatlimab in combination with 14C12H1L1(hG1TM) could promote IL-2 secretion, with comparable activities.

### Experimental Example 6: Assays for Biological Activity of Anti-LAG Antibodies in Blocking Interaction between LAG-3 and MHC-II (Reporter Gene Method)

Jurkat-NFAT-PD1-LAG3 cells and Raji cells were used as a reporter gene system. After a superantigen SEE was added, a TCR-NFAT signaling pathway was activated to induce the expression of luciferase. The LAG-3 on the Jurkat cells was bound to the MHC-II on the Raji cells, such that the NFAT signaling pathway was inhibited, and the expression of luciferase was down-regulated. The antibody, by specifically binding to LAG-3, relieved the inhibition and up-regulated the expression of luciferase.

Jurkat-NFAT-PD1-LAG3 cells and Raji cells (purchased from the Cell Resource Center, Shanghai Institutes for Biological Sciences, Chinese Academy of Sciences, Cat. No. TCHu 44) were collected and centrifuged at 110× g for 5 min, followed by removal of the supernatant. The cells were then resuspended in a 1640 + 10% FBS medium and counted. The Jurkat-NFAT-PD1-LAG3 cells were seeded into a black-bottom 96-well plate (Corning, Model No. 3916) at 10⁵ cells/well (30 µL/well); according to the experimental design, antibodies (at final concentrations of 0.3 nM, 3 nM, and 300 nM, respectively) were added at 10 µL/well, and the mixture was pre-incubated at 37 °C in a 5% CO₂ incubator for 30 min. Meanwhile, SEE (Staphylococcal Enterotoxins E, purchased from Toxin Technology, Cat. No. ET404) (at a final concentration of 0.05 ng/mL) was added to the Raji cells, and the mixture was incubated at 37 °C in a 5% CO₂ incubator for 30 min. After 30 min, the SEE-treated Raji cells were added into the 96-well plate containing Jurkat-NFAT-PD1-LAG3 cells described above at 2×10⁴ cells/well (40 µL/well), with the final volume of each well being 80 µL. The mixture was mixed well and incubated at 37 °C in a 5% CO₂ incubator for 6 h. After incubation, the culture plate was taken out and allowed to equilibrate to room temperature. Bright-Glo^{™}Luciferase Assay System (purchased from Promega, Cat. No. E2650) was added at 80 µL/well, and the mixture was incubated in the dark for 2 min. Then the RLU values were read. The isotype control hIgG1DM was constructed by Akeso Biopharma Inc. with a Batch No. 20181107; the isotype control hG4WT was constructed by Akeso Biopharma Inc. with a Batch No. 20190910.

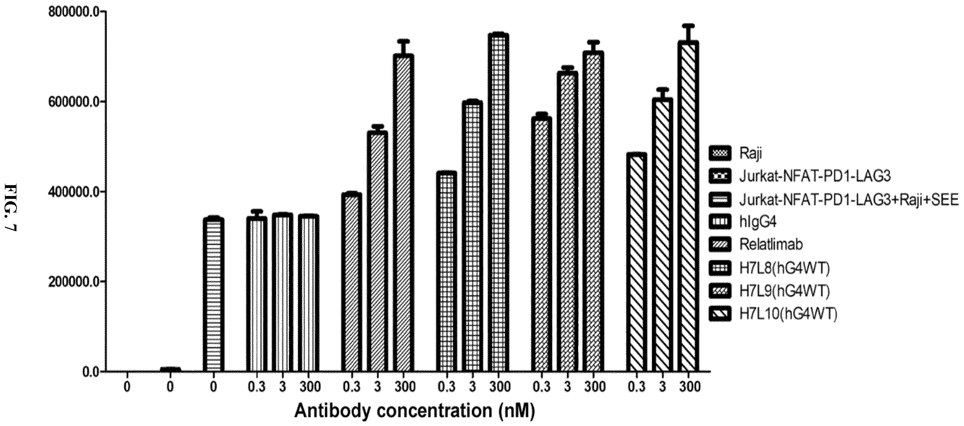

As shown in FIG. 7, the anti-LAG antibodies H7L8(hG4WT), H7L9(hG4WT) and H7L10(hG4WT), and the positive control antibody Relatlimab could block the interaction between LAG-3 and MHC-II to up-regulate the expression of luciferase, and the activities of the anti-LAG antibodies H7L8(hG4WT), H7L9(hG4WT) and H7L10(hG4WT) were all superior to that of the control antibody Relatlimab.

Although specific embodiments of the present invention have been described in detail, those skilled in the art will appreciate that various modifications and substitutions can be made to those details according to all the teachings that have been disclosed, and these changes shall all fall within the protection scope of the present invention. The full scope of the present invention is given by the appended claims and any equivalent thereof.

## Claims

1. An anti-LAG3 antibody or an antigen-binding fragment thereof, comprising a heavy chain variable region and a light chain variable region, wherein
the heavy chain variable region comprises HCDR1-HCDR3 having amino acid sequences set forth in SEQ ID NOs: 9-11, respectively, and the light chain variable region comprises LCDR1-LCDR3 having amino acid sequences set forth in SEQ ID NOs: 12-14, respectively; the heavy chain variable region comprises HCDR1-HCDR3 having amino acid sequences set forth in SEQ ID NOs: 9-11, respectively, and the light chain variable region comprises LCDR1-LCDR3 having amino acid sequences set forth in SEQ ID NO: 12, SEQ ID NO: 15 and SEQ ID NO: 16, respectively;
or
the heavy chain variable region comprises HCDR1-HCDR3 having amino acid sequences set forth in SEQ ID NOs: 9-11, respectively, and the light chain variable region comprises LCDR1-LCDR3 having amino acid sequences set forth in SEQ ID NO: 17, SEQ ID NO: 15 and SEQ ID NO: 14, respectively.

2. The antibody or the antigen-binding fragment thereof according to claim 1, wherein
the heavy chain variable region of the antibody has an amino acid sequence set forth in SEQ ID NO: 2, and the light chain variable region of the antibody has an amino acid sequence set forth in SEQ ID NO: 4;
the heavy chain variable region of the antibody has an amino acid sequence set forth in SEQ ID NO: 2, and the light chain variable region of the antibody has an amino acid sequence set forth in SEQ ID NO: 6;
or
the heavy chain variable region of the antibody has an amino acid sequence set forth in SEQ ID NO: 2, and the light chain variable region of the antibody has an amino acid sequence set forth in SEQ ID NO: 8.

3. The antibody or the antigen-binding fragment thereof according to any one of claims 1 to 2, wherein the antibody or the antigen-binding fragment thereof is selected from a Fab, a Fab', an F(ab')₂, an Fd, an Fv, a dAb, a complementarity determining region fragment, a single chain fragment variable, a humanized antibody, or a chimeric antibody.

4. The antibody or the antigen-binding fragment thereof according to any one of claims 1 to 3, wherein the antibody binds to human LAG3-mFc with an EC₅₀ value of less than 0.2 nM, such as less than 0.15 nM, less than 0.1 nM, less than 0.08 nM, 0.06 nM, or less than 0.05 nM, or less; preferably, the EC₅₀ value is determined by indirect ELISA.

5. The antibody or the antigen-binding fragment thereof according to any one of claims 1 to 4, wherein
the antibody comprises a non-CDR region derived from a species other than murine, such as from a human antibody.

6. The antibody or the antigen-binding fragment thereof according to any one of claims 1 to 5, wherein
the antibody comprises a constant region derived from a human antibody;
preferably, the constant region of the antibody is selected from constant regions of human IgG1, IgG2, IgG3 or IgG4.

7. The antibody or the antigen-binding fragment thereof according to any one of claims 1 to 6, wherein
a heavy chain constant region of the anti-LAG3 antibody is Ig gamma-1 chain C region (e.g., as set forth in SEQ ID NO: 18) or Ig gamma-4 chain C region (e.g., as set forth in SEQ ID NO: 20), and a light chain constant region of the anti-LAG3 antibody is Ig kappa chain C region (e.g., as set forth in SEQ ID NO: 19).

8. An antibody-drug conjugate, comprising an antibody or an antigen-binding fragment thereof and a small molecule drug, wherein the antibody or the antigen-binding fragment thereof is the antibody or the antigen-binding fragment thereof according to any one of claims 1 to 7; preferably, the small molecule drug is a small molecule cytotoxic drug; and more preferably, the small molecule drug is an anti-tumor chemotherapeutic drug.

9. The antibody-drug conjugate according to claim 8, wherein the antibody or the antigen-binding fragment thereof is linked to the small molecule drug via a linker; for example, the linker is a hydrazone bond, a disulfide bond, or a peptide bond.

10. The antibody-drug conjugate according to claim 8 or 9, wherein the molar ratio of the antibody or the antigen-binding fragment thereof to the small molecule drug is 1:(2-4).

11. An isolated nucleic acid molecule, encoding the anti-LAG3 antibody according to any one of claims 1 to 7.

12. A recombinant vector, comprising the isolated nucleic acid molecule according to claim 11.

13. A host cell, comprising the isolated nucleic acid molecule according to claim 11 or the recombinant vector according to claim 12.

14. A method for preparing the antibody or the antigen-binding fragment thereof according to any one of claims 1 to 7, comprising: culturing the host cell according to claim 13 in a suitable condition, and isolating the antibody or the antigen-binding fragment thereof from the cell cultures.

15. A pharmaceutical composition, comprising the antibody or the antigen-binding fragment thereof according to any one of claims 1 to 7, or the antibody-drug conjugate according to any one of claims 8 to 10, wherein optionally, the pharmaceutical composition further comprises a pharmaceutically acceptable auxiliary material.

16. Use of the antibody or the antigen-binding fragment thereof according to any one of claims 1 to 7, or the antibody-drug conjugate according to any one of claims 8 to 10 in the preparation of a medicament for treating and/or preventing a tumor or anemia, wherein preferably, the tumor is selected from one or more of ovarian cancer, esophageal cancer, melanoma, a hematological malignancy, glioblastoma, renal cell carcinoma, lung cancer, prostate cancer, bladder cancer, colon cancer, rectal cancer, liver cancer, gastrointestinal cancer, breast cancer, brain cancer, pancreatic cancer, thyroid cancer, head and neck cancer, and kidney cancer;
preferably, the lung cancer is non-small cell lung cancer;
preferably, the hematological malignancy is leukemia;
preferably, the esophageal cancer is esophageal squamous cancer.

17. The antibody or the antigen-binding fragment thereof according to any one of claims 1 to 7 or the antibody-drug conjugate according to any one of claims 8 to 10 for use in treating and/or preventing a tumor or anemia, wherein
preferably, the tumor is selected from one or more of ovarian cancer, esophageal cancer, melanoma, a hematological malignancy, glioblastoma, renal cell carcinoma, lung cancer, prostate cancer, bladder cancer, colon cancer, rectal cancer, liver cancer, gastrointestinal cancer, breast cancer, brain cancer, pancreatic cancer, thyroid cancer, head and neck cancer, and kidney cancer;
preferably, the lung cancer is non-small cell lung cancer;
preferably, the hematological malignancy is leukemia;
preferably, the esophageal cancer is esophageal squamous cancer.

18. A method for treating and/or preventing a tumor or anemia, comprising a step of administering to a subject in need an effective amount of the antibody or the antigen-binding fragment thereof according to any one of claims 1 to 7 or the antibody-drug conjugate according to any one of claims 8 to 10, wherein
preferably, the tumor is selected from one or more of ovarian cancer, esophageal cancer, melanoma, a hematological malignancy, glioblastoma, renal cell carcinoma, lung cancer, prostate cancer, bladder cancer, colon cancer, rectal cancer, liver cancer, gastrointestinal cancer, breast cancer, brain cancer, pancreatic cancer, thyroid cancer, head and neck cancer, and kidney cancer;
preferably, the lung cancer is non-small cell lung cancer;
preferably, the hematological malignancy is leukemia;
preferably, the esophageal cancer is esophageal squamous cancer.
